# EUROPEAN PATENT SPECIFICATION

(11) **EP 4 472 589 B1**
(45) Date of publication and mention of the grant of the patent: **04.03.2026**
(21) Application number: 23702258.7
(22) Date of filing: 25.01.2023
(51) Int. Cl.: A61F 9/00, B65D 47/18

(54) **DROPPER FOR DISPENSING A PHARMACEUTICAL LIQUID**
TROPFER ZUR ABGABE EINER PHARMAZEUTISCHEN FLÜSSIGKEIT
COMPTE-GOUTTES POUR DISTRIBUER UN LIQUIDE PHARMACEUTIQUE

(30) Priority: 02.02.2022 DE 102022102476
(43) Date of publication of application: 11.12.2024
(73) Proprietor: Silgan Dispensing Systems Hemer GmbH, 58675 Hemer (DE)
(72) Inventor: CIEZAREK, Marek, 44287 Dortmund (DE)
(74) Representative: Sparing Röhl Henseler
(86) International application number: PCT/EP2023/051738
(87) International publication number: WO 2023/148055

(56) References cited:
- EP-A1- 2 598 409
- EP-A1- 3 858 303
- DE-A1- 102018 124 067
- DE-B3- 102012 214 426

## Description

The invention relates to a dropper for dispensing a pharmaceutical liquid according to the preamble of claim 1. Such droppers are preferably designed as a multidose dropper.

From EP 3 858 303 A1 a dropper for dispensing a pharmaceutical liquid in drop form having a liquid reservoir and a dropper head is known according to the preamble of claim 1.

A liquid dispenser for dispensing drops is known from DE 10 2012 214 426 B3. Such a liquid dispenser comprises a housing, a liquid reservoir, a discharge orifice through which liquid can be discharged from the liquid reservoir, and a drop formation surface that surrounds the discharge orifice outside and enables a drop to be formed by delivering liquid as a drop from the drop formation surface. The drop separates from the dispenser when it is of sufficient size to release the majority of the discharged liquid in the form of a main drop.

With drop dispensers, there is the problem that when a drop, the main drop, is dispensed, a residue, the so-called residual drop, usually remains on the drop formation surface, which subsequently either dries on the drop formation surface or is sucked back into the drop dispenser. This residual drop can hardly be avoided technically and represents a hazard, as it can cause contamination both on the drop formation surface and in the liquid reservoir in the event of multiple use.

**In** the case of drop dispensers that do not have an outlet valve associated with the discharge orifice, this residual drop is consequently drawn back into the bottle by the negative pressure previously developed in the liquid reservoir. **In** drop dispensers with a pressure-dependent opening outlet valve, on the other hand, the return path into the drop dispenser for the residual drop is closed after the end of the discharge process, so that the residual drop remains on the outside of the discharge orifice. This is problematic because, as already explained, this residual drop can become a carrier of contamination.

**It** is therefore desirable to minimize the volume of the residual drop as far as possible so that contamination is kept to a minimum, especially during drying on the drop formation surface. A smaller residual drop leads to faster drying of the same and thus to a reduced risk of contamination.

A change in the shape of the drop formation surface makes it possible to significantly reduce the residual drop in a simple design way. **It** has been shown that a shape deviating from the usual circular shape of the outside surface unlikely influences the amount of liquid of the main drop to be dispensed, when having a similar size of the drop formation surface, but significantly reduces the size of the residual drop remaining on the drop formation surface after the main drop has been dispensed.

Preferably, the shape of the drop formation surface deviates significantly from the circular shape, with at least sections of the outside surface forming indentations that are concavely curved inwards and thus towards the discharge opening, which results in a particularly small residual droplet. The residual drop remaining on the drop formation surface is then only about half as large as a residual drop which would remain on a circular drop formation surface of the same area.

The dosage of medication that is applied in drop form is usually done by the number of drops, so that furthermore the achievement of a more constant size of the delivered main drop is advantageous.

DE 10 2011 083 355 84 discloses a drop dispenser which permits the discharge of drops having substantially constant size or liquid volume, and shortens the drying time of the remaining residual drop. For this purpose, the drop formation surface on the outside of the discharge orifice, relative to an orientation of the dropper with the discharge orifice pointing vertically downward, has a shape tapering downward and toward the discharge orifice. In addition, said drop formation surface is designed to be hydrophilic. The combination of the tapered and hydrophilic designed droplet formation surface surrounding the discharge orifice has been proven to be advantageous, particularly with regard to the drying time of the residual droplet.

In order to be able to produce drops of substantially constant size, even when deviating from a vertical orientation, it can be included that the drop formation surface is formed by an outer surface of a drop formation body which has the shape of a spherical calotte. So, it is possible to ensure that tilting the drop dispenser relative to a vertical orientation has no effect on the size of the drop.

All these solutions are based on the assumption that it is advantageous to provide for the residual drop, remaining on the dispenser, an increased area as a drop formation surface, if by a valve pin the outlet orifice is closed in the closed condition of the dropper. The valve pin is part of an outlet valve, which opens and closes depending on the liquid pressure inside the drop dispenser.

Droppers that do not have an outlet valve with a valve pin associated with the outlet orifice can have pressure-deformable elastic dispensing ends. From EP 0 222 944 A1, for example, a dropper with a tubular dropper head is known, the outlet orifice of which is designed as a beak valve.

To improve the formation of individual drops of such dropper, it is known from DE 6933320 U1 to make the drop formation independent of the pressing pressure exerted on the actuating chamber. For this purpose, the interior of a spout-like dispensing tip consists of a cylindrical longitudinal bore divided by sections forming expansion chambers arranged at an axial distance from each other for slowing down the flow rate.

In the case of particularly low-viscosity liquids, for example, a total of three expansion chambers can be provided, while one expansion chamber can be sufficient for relatively high-viscosity liquids. Exact dosing is achieved. However, since the liquid remaining in the spout is also drawn back perfectly when the actuation chamber is depressurized, the risk of contamination is high. According to US 6,105,828, an improvement is seen in the fact that the inner diameter transitions are rounded.

A dropper is known from WO 84/00707 A1, in which the dropper head has a capillary orifice with a conical portion enabling a drop. The length and diameter of the capillary tube determine the speed of drop formation. The conical portion then determines the drop size, with the drop size increasing as the area of the drop formation surface on the cone increases. The cone angle is preferably 50° to 64°. From EP 0 594 490 B1 it is known to form such a conical portion enabling a drop having a star-shaped discharge orifice comprising at least three arms.

From DE 20 2013 010 261 U1 is known an ophthalmic product for multidose use, which comprises a container with an integrated bacteria protection system and has a dispensing tip for drops. In summary, it is noted that several constructions of drop dispensing tips can be distinguished. The simplest design is a nozzle with a small orifice for fluid passage; dropper tips with a straight, elongated, cylindrical channel of uniform cross-section and a narrower internal orifice; tips with a conical outward channel under a cylindrical recessed channel. Changes in eye dropper tip dimensions can significantly affect drop volumes.

The flow of liquid through the dropper tip and the drop size depends on the inner opening and the outer diameter of the outlet. The inner diameter of the outlet of the dropper tip is preferably constant. In such a system, the eye drop size increases linearly with the outer diameter. Drop bottle tips according to the prior art control the size of the drops by controlling the inner diameter relative to the outer diameter. Preferably, the dispensing tip should have the following dimensions: The ratio of the inner diameter to the outer diameter of the dispensing tip is from 1:1 to 1:6, most preferably from 1:1 to 1:3, with the outer diameter corresponding to the diameter of the opening at the tip of the nozzle where the drop exits the container and the inner diameter corresponding to the diameter inside the nozzle that guides the drop to the opening where the drop exits the receptacle.

It should be noted with regard to ophthalmic products that the volume of a human tear is on average 7 µl. The conjunctival sac can hold 20-30 µl of fluid without overflowing onto the cheek. However, the average drop size of commercial ophthalmic medications is 39 µl. The excess fluid overflows onto the cheeks or drains into the nasolacrimal system, where it can cause undesirable side effects. In order to obtain eye drops with ideal volume, dropper tips with smaller dimensions are required. It is therefore known, to reduce the drop volume to 22 µl to 31 µl by taking into account the influencing factors of viscosity, surface tension and dimensions of the dispensing tip.

However, the problem of residual drops has proven to be a disadvantage. As the outer diameter of the dispensing tip increases, the drop formation area and its ability to dry a residual drop increases. However, as the droplet size increases, so does the residual droplet and thus the risk of contamination.

It is therefore advantageous to provide a dropper that is designed for dispensing individual drops of defined size and thereby reliably prevents contamination of liquid entering from the outside.

According to embodiments of the invention, a dropper is provided that uses the known physical phenomenon of dripping of liquids at nozzles or capillaries. The drop size produced depends on the capillary or nozzle diameter as well as the effective surface tension and density of the liquid.

If the liquid in the earth's gravitational field flows through a vertically arranged capillary or nozzle at a low inflow velocity, a liquid drop adhering to the capillary or nozzle is initially formed as a result of the effective surface tension. Its mass successively increases due to the adding of liquid. As soon as the weight force of the adhering drop becomes greater than the surface tension force holding the drop to the capillary or nozzle, the drop detaches, leaving a liquid meniscus on the nozzle or capillary.

The invention achieves that when the respective drop detaches from the orifice in the earth's gravitational field, the drop undergoes a deformation in this acceleration field and constricts behind the orifice in such a way that substantially the entire liquid is captured by the detaching drop, reducing the mass remaining at the orifice, i.e. the volume of the residual drop.

It is to the merit of the inventors of the present application to have recognized that a discharge orifice having an outlet deviating from a circular shape associated with an inner circumferential drip shoulder comprising circumferentially different radial widths, influences the drop formation process. The menisci formed by the liquids in capillaries depend on the radius of the capillary. According to the invention, modified metric characteristics are formed, since the circumferential drip shoulder is formed with different radial widths with respect to the center point and the respective deviation is preferably executed centrally with respect to the annular opening of the inlet contour. The shaping of the inner cross-section of the orifice in the area of the outlet contour according to the invention together with the drip shoulder reduces the volume of the residual droplet. This advantage is also particularly apparent for more highly viscous liquids and when drops are dispensed from the dropper tip at an angle of 90° to the horizontal, where the drop weight per se is greater than when the delivery angle is changed to 45°.

Repeated delivery of drops with a substantially constant volume, i.e. the main drop, is made possible while reducing the volume of the residual drop. The use of preservative-free medical fluids and the accuracy of the administered dose is thus significantly improved.

The circumferential inner drip shoulder may be formed as a planar surface transverse to the axial axis of the drip dispenser. Alternatively, the circumferential drip shoulder can be formed entirely or in sections by a curved surface section.

The shape of the outlet contour can be selected, for example, as an ellipse or polygon, with the numerical eccentricity preferably in the range 0.01 to 0.8. A drip step formed as a drip shoulder can be between 1 and 7 mm².

To provide drop size and drop size variability the wall thickness of the dispensing tip in the area of the outlet opening between the inner wall and outer wall can be in the range of 1:1 to 1:6, especially 1:1 to 1:3, as known.

For overhead operation, it can also be provided that a venting path outlet of a pressure compensation channel into the reservoir can be closed in the manner of a non-return valve via a sealing device provided on the discharge head. The sealing device can, for example, be a sealing disc curved concavely towards the inside of the storage container, the edge of which is in sealing contact.

Further embodiments and advantages of the invention are described in the following description and the claims.

The invention is explained in more detail below with reference to the embodiments shown in the accompanying figures, in which:
Fig. 1 schematically shows a longitudinal section of a dropper head of a dropper dispenser according to an embodiment of the invention;
Fig. 2 schematically shows a cross-section of the dropper head according to Fig. 1 with an enlarged view of an inlet contour;
Figs. 3a and 3b schematically show a perspective view and an associated cross-section of the dropper head according to Fig. 1 with an enlarged view of an outlet contour without a valve body;
Fig. 4 shows a schematic perspective view of the dropper head according to Fig. 1 with illustration of the outlet contour;
Fig. 5 schematically shows a perspective view of a dropper head of a dropper dispenser according to another embodiment;
Fig. 6 schematically shows a perspective view of a dropper head of a dropper dispenser according to an alternate embodiment;

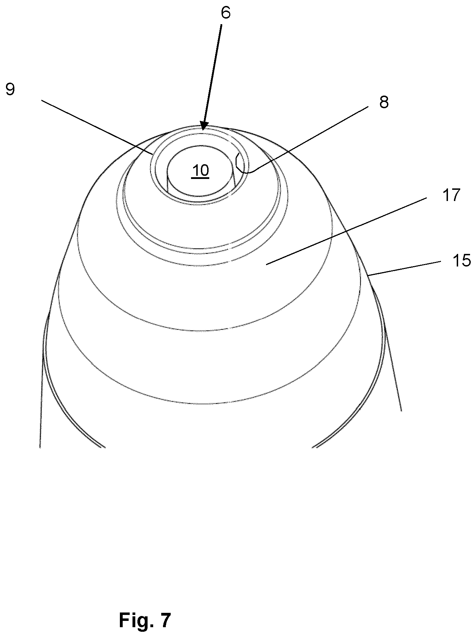

Fig. 7 schematically shows a perspective view of a dropper attachment of a dropper dispenser according to an alternate embodiment;
Fig. 8 shows a longitudinal section of a dropper head attached to a neck extension of a liquid container of a dropper dispenser according to an embodiment;
Fig. 9 is an overview of different drop sizes at different viscosities and the resulting average residual drops obtained from embodiments of the invention; and
Fig. 10 schematically shows a longitudinal section of a dropper head of a dropper dispenser according to an alternate embodiment.

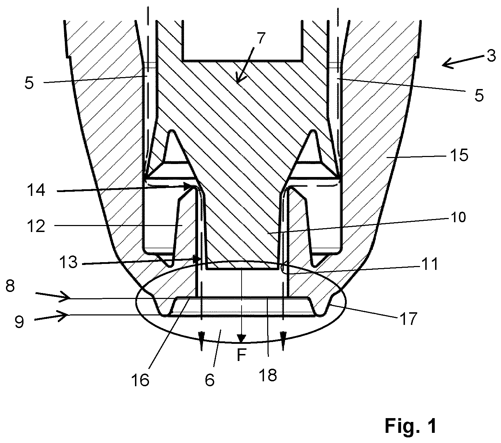

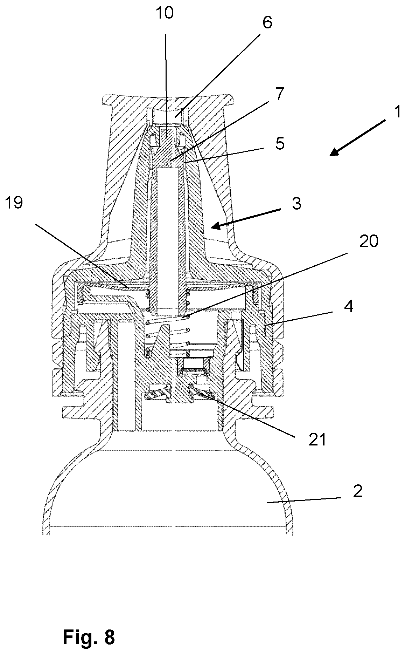

As shown in Fig. 1 and Fig. 8, the invention relates to a dropper dispenser 1 for dispensing a pharmaceutical liquid drop after drop. This dropper dispenser 1 has a liquid reservoir 2, in particular a bottle or flask, for receiving the liquid medium and a dispensing head which can be fixed to the liquid reservoir 2 and is designed as a dropper head 3. The liquid reservoir 2 is preferably bottle-shaped and can manually be compressed so that the resulting increase in pressure in the liquid reservoir 2 causes a medium stroke or movement of the liquid medium out of the reservoir 2. The liquid reservoir 2 is surmounted by the dropper head 3.

In principle, various types of delivery mechanisms are possible for this purpose. Preferably, it is provided that the walls of the liquid reservoir or a housing surrounding the liquid reservoir are deformable, so that the volume of the liquid reservoir can be reduced by direct application of force to the walls or housing, which acts as a squeeze bottle. However, other mechanisms such as a piston pump are also conceivable and encompassed by the invention.

The dropper head 3 is preferably attached to a neck of the liquid reservoir 2 via a closure 4, for example a snap closure. The dropper dispenser 1 relates, for example, to the dispensing of eye drops.

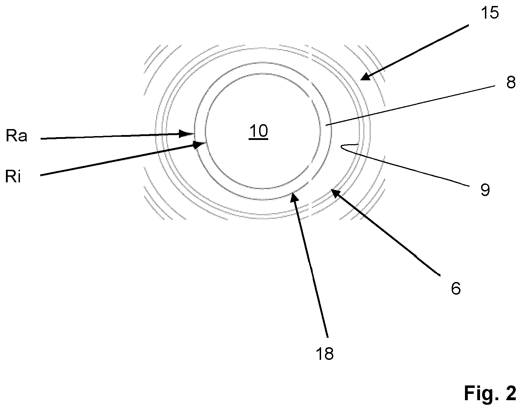

The dropper head 3 comprises a channel 5 that ends in an outlet orifice 6. The outlet orifice 6 provides a nozzle, and an outlet valve 7 is associated with the outlet orifice 6. The outlet orifice 6 has a capillary orifice shape having an inlet contour 8 and an outlet contour 9. The inlet contour 8 provides an inner annular opening 18 on the outlet side of a central valve body 10 of the outlet valve 7 for the fluid to be conveyed through outlet orifice 6. Fig. 1 shows the fluid path F and Fig.2 shows a radius Ri as a circumferential jacket surface of a cylindrical valve body 10 opposite a radius Ra as an inner diameter of a capillary 12 into which the channel 5 opens. A stabilization zone 13 may be formed between the valve body 10 and the inner wall 11 of the capillary 12. Furthermore, a microbiological closure 14 can be provided on the inlet side of the capillary 12. The capillary 12 is installed in a material 15 which surrounds the outlet orifice 6 on the outside. The surface provided on the outside of the outlet orifice 6, which is wetted during the discharge of liquid and defines an outer drop formation surface 17 as an outer boundary contour, is explained below.

Both the cross-section of the cavity located in the capillary 12 and the outer cross-section of the capillary 12 are preferably round in shape. However, the cross-section may also have another shape, for example an oval, square, rectangular or polygonal shape.

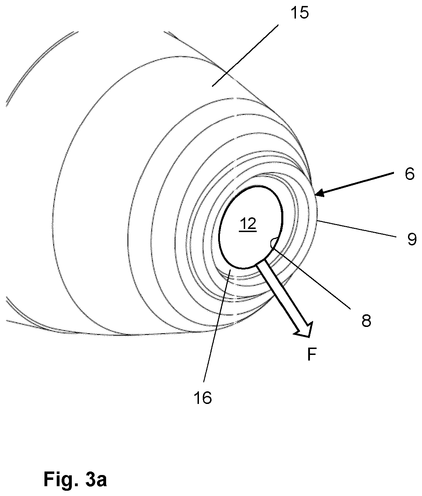

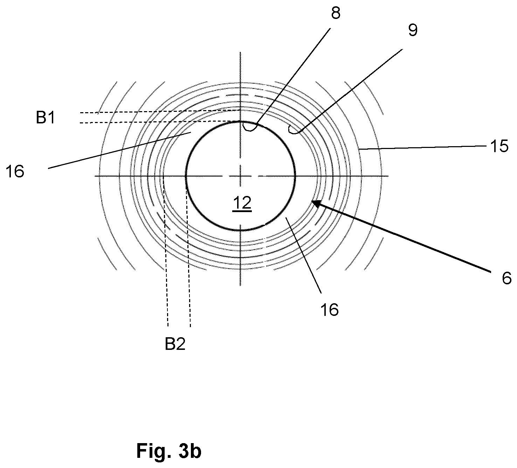

As shown in particular in Figs. 3a and 3b, the inner cross-section of the capillary 12 of the outlet orifice 6 is divided by at least one radially outwardly extending annular shoulder 16, opposite which the outlet contour 9 extends with a shape deviating from a circular shape. As a result, the annular shoulder 16 forms an inner-walled drip stage with circumferentially different radial widths B1, B2 relative to the annular orifice of the inlet contour 8. The width B1 is, for example, the smallest width and B2 is, for example, the largest width and the intermediate widths lie between this minimum value and the maximum value. The inner cross-section of the capillary 12 widens through the shoulder 16.

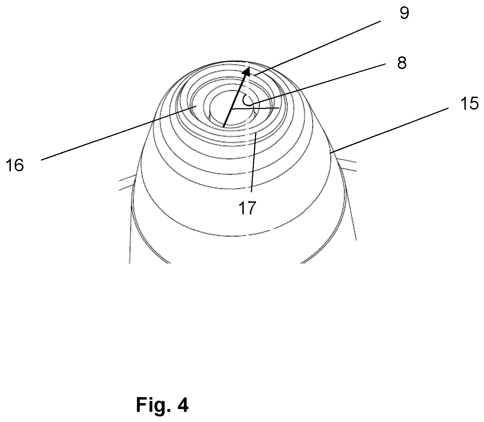

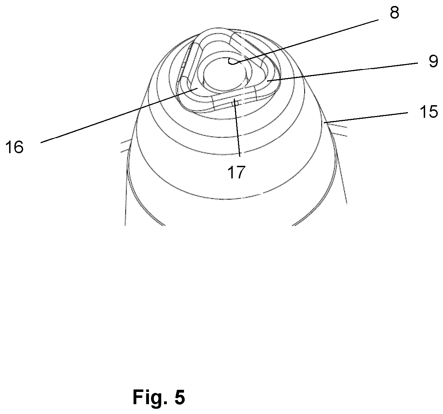

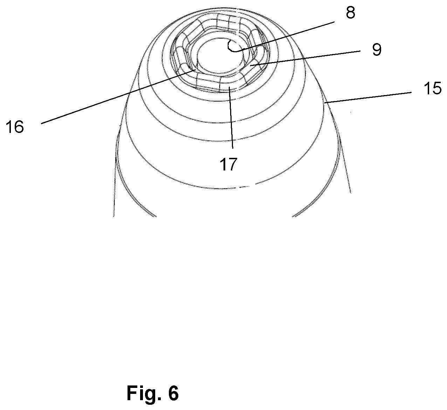

The annular shoulder 16 preferably is positioned centrally opposite a circular orifice of the inlet contour 8. The shape of the outlet contour 9, which deviates from the circular shape, can be in the form of an ellipse, as shown in Fig. 4, or a polygon, as shown in Figs. 5 and 6. The numerical eccentricity of the outlet contour 9 in combination with the annular shoulder 16 may be in the range of 0.01 to 0.8. The drip stage formed by the annular shoulder 16 may occupy an area range of 1 to 7 mm². To determine the drop size, a wall thickness of a dispensing tip embodying the outlet orifice 6, in this case of the outlet contour 9, in the area of an outer contour between the inner wall and the outer wall can be in the range of 1:1 to 1:6, in particular 1:1 to 1:3.

On the outside, the outlet contour 9 includes a drop-forming surface 17, which is formed by an outer side of a dropper head acting as a drop-forming body. In a use position, the outlet orifice 6 with the outlet contour 9 is directed downward, for example. Tilting the dropper dispenser 1 with respect to a vertical direction by up to 25° usually has no effect on the size of the droplet.

The drop forming surface 17 may have a downwardly tapering shape in an outer region relative to an orientation of the dropper dispenser 1 with the discharge orifice 6 facing vertically downward.

The drop formation area 17 may be limited on the outside by a border edge. This border edge can further improve the drop formation process.

The drop formation surface 17 can be concave or convex.

The outlet orifice 6 is preferably associated with the outlet valve 7 including a preloaded valve body 10. As Fig. 8 shows, the valve body 10 can be force-loaded in the direction of the discharge orifice 6 by a valve spring 20. The valve spring 20 is located outside the fluid path F. A pressure equalization channel is further sealed with respect to the liquid reservoir 2 and its interior by a sealing device. The sealing device can be designed as a sealing disk 21 or sealing plug.

When a force is applied to the liquid reservoir 2, the liquid is forced through the outlet orifice 6 with the outlet valve 7 slightly open and is received in the capillary outlet form. As a result, the liquid passes through the annular orifice 18 of the inlet contour 8 and enters the outlet contour 9 through the cross-sectional orifice of the shoulder 16. In Fig. 1, the flow path F is drawn.

Thus, according to the invention, the discharged liquid is received in the area of the outlet orifice 6 and forms a drop there in the interaction of the inlet contour 8 and outlet contour 9, which detaches from the dropper dispenser 1 after reaching a certain size.

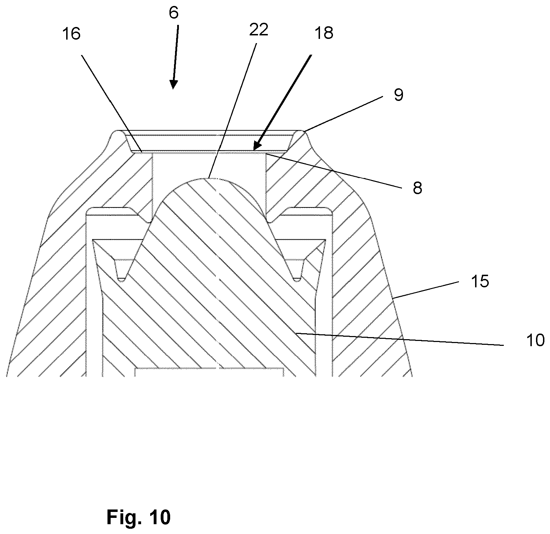

The center valve body 10 may be formed as a flat surface on the head side, as shown in Figs. 1 and 10. Alternatively, a curved surface may be formed as the end face 22. The curved surface may be dome-shaped. The end surface 22 may be formed as a curved surface that in addition is hydrophilic.

According to a further embodiment, at least two annular shoulders 16 arranged at an axial distance from each other may be provided.

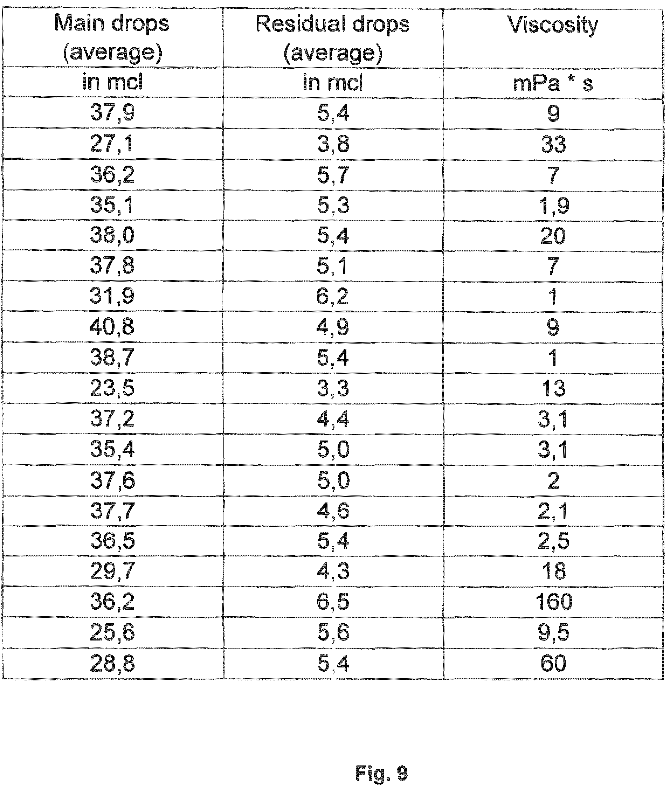

Fig. 9 shows an overview of the dispensing of main drops for various pharmaceutical liquids with different viscosities and the associated residual drops. The main drops are mostly in the preferred range up to 39 µl and the viscosities vary between 1 and 160 mPas. About 80% of the remaining residual drops are in a volume range less than or equal to 5.4 µl, which is advantageous for residual drops, for viscosities as high as 60 mPas.

The drop dispenser 1 according to Fig. 8 is preferably equipped with a valve spring 20 being relatively weak. Combined with a relatively large flat pressurization surface 19, the outlet opening 6 already opens at a relatively low overpressure of, for example, 0.2 to 0.5 bar. The medium then flows through the outlet orifice 6 in only a slightly pressurized state, which serves the intended growing of a drop at the outlet orifice 6. These functional elements are described in detail in EP 3 576 825 B1, to which reference is hereby made.

Finally, the solution according to the invention can also be combined with solutions known from the prior art to design the dropper 1 in liquid-carrying areas with bactericidal, i.e. bacteria killing, or bacteriostatic, or bacteria growth preventing, surfaces in order to eliminate or avoid any contamination that may occur.

The material for the dropper can be polyethylene (PE), a thermoplastic elastomer (TPE) or polypropylene (PP).

Finally, a protective cap can be placed on the dropper in a known manner.

The dropper 1 can be used for any type of fluid or liquid medium. The medium can be preservative-free formulations. This preservative-free, multidose ophthalmic dispenser according to the invention ensures an easy, consumer-friendly experience through exceptional ergonomics for precision and accuracy down to the drop.

## Claims

1. Dropper (1) for dispensing a pharmaceutical liquid forming a drop, comprising a liquid reservoir (2) and a dropper head (3), wherein the dropper head (3) includes a channel (5) ending in an outlet orifice (6), wherein an outlet valve (7) is associated with the outlet orifice (6), and the outlet orifice (6) has a capillary orifice shape with an inlet contour (8) and an outlet contour (9) and the inlet contour (8) forms an inner annular orifice (18) on the outlet side of a central valve body (10) of the outlet valve (7), wherein the capillary (12) of the outlet orifice (6) is divided by at least one radially outwardly extending annular shoulder (16), **characterized in that** with respect to said annular shoulder (16) the outlet contour (9) extends with a shape deviating from a circular shape, and the annular shoulder (16) forms an inner-walled drip step with circumferentially different radial widths (B1, B2) with respect to the annular opening (18) in the inlet contour (8).

2. Dropper (1) according to claim 1, **characterized in that** the annular shoulder (16) is formed centrally opposite a circular opening of said round capillary (12) of the inlet contour (8).

3. Dropper (1) according to claim 1 or 2, **characterized in that** the shaping of the outlet contour (9) deviating from the circular shape is designed in the manner of an ellipse or a polygon.

4. Dropper (1) according to any one of claims 1 to 3, **characterized in that** the numerical eccentricity of the outlet contour (9) is in the range 0.01 to 0.8.

5. Dropper (1) according to any one of claims 1 to 4, **characterized in that** the formed drip stage occupies an area range of 1 to 7 mm²

6. Dropper (1) according to any one of claims 1 to 5, **characterized in that** a wall thickness of a dispensing tip receiving the outlet orifice (6) in the region of the outer contour between the inner wall and the outer wall is in the range 1:1 to 1:6, in particular 1:1 to 1:3.

7. Dropper (1) according to any one of claims 1 to 6, **characterized in that** the central valve body (10) is designed as a curved surface on the head side.

8. Dropper (1) according to claim 7, **characterized in that** the curved surface is dome-shaped.

9. Dropper (1) according to claim 7 or 8, **characterized in that** the curved surface is hydrophilic.

10. Dropper (1) according to any one of claims 1 to 9, **characterized in that** the outlet contour (9) has on the outside a drop forming surface (17) formed by an outside of a drop forming body of the dropper head (3).

11. Dropper (1) according to any one of claims 1 to 10, **characterized in that** the drop forming surface (17) has a downwardly tapering shape in an outer region relative to an orientation of the dropper dispenser (1) with the orifice (6) pointing vertically downward.

12. Dropper (1) according to claim 10 or 11, **characterized in that** the drop formation surface (17) is delimited on the outside by a boundary edge.

13. Dropper (1) according to any one of claims 10 to 12, **characterized in that** the drop formation surface (17) is concave or convex.

14. Dropper (1) according to any one of claims 1 to 13, **characterized in that** at least two annular shoulders (16) arranged at an axial distance from one another are provided.

15. Dropper (1) according to any one of claims 1 to 14, **characterized in that** the outlet valve (7) associated with the outlet orifice (6) is formed with a preloaded valve body (10).

16. Dropper (1) according to claim 15, **characterized in that** the valve body (10) is force-loaded by means of a valve spring (20) in the direction of the outlet opening (6).

17. Dropper (1) according to any one of claims 1 to 16, **characterized in that** the liquid reservoir (2) is designed as a squeeze bottle and the dropper head (3) is fixed to a neck attachment of the squeeze bottle.

18. Dropper (1) according to any one of claims 1 to 17, **characterized in that** a pressure equalization channel (D) is sealed with respect to the liquid reservoir (2) and its interior by means of a sealing device.

19. Dropper (1) according to claim 18, **characterized in that** the sealing device is designed as a sealing disc (21) or sealing plug.

20. Dropper (1) according to any one of claims 1 to 19, **characterized in that** the dropper head (3) comprises, as an external component, a dispensing adapter having a tubular extension which is a drop adapter for dispensing eye drops.

21. Dropper (1) according to any one of claim 1 to 20, **characterized in that** it has a liquid reservoir (2) for receiving the liquid medium for multiple **use.**

## Patentansprüche

1. Tropfenspender (1) zur Abgabe einer pharmazeutischen Flüssigkeit in Tropfenform mit einem Flüssigkeitsspeicher (2) und mit einem Tropfaufsatz (3), wobei der Tropfaufsatz (3) einen in eine Auslassöffnung (6) mündenden Kanal (5) aufweist, wobei der Auslassöffnung (6) ein Auslassventil (7) zugeordnet ist und die Auslassöffnung (6) eine kapillare Mündungsform mit einer Einlasskontur (8) und einer Auslasskontur (9) besitzt und die Einlasskontur (8) eine innere ringförmige Öffnung (18) abgabeseitig eines mittleren Ventilkörpers (10) des Auslassventils (7) bildet, die Kapillare (12) der Auslassöffnung (6) durch mindestens eine radial nach außen sich erstreckende ringförmige Schulter (16) unterteilt ist, **dadurch gekennzeichnet, dass** gegenüber der ringförmigen Schulter (16) sich die Auslasskontur (9) mit einer von einer Kreisform abweichenden Formgebung erstreckt, und die ringförmige Schulter (16) eine innenwandige Abtropfstufe mit umfänglich unterschiedlicher radialer Breite (B1, B2) gegenüber der ringförmigen Öffnung (18) in der Einlasskontur (8) bildet.

2. Tropfenspender (1) nach Anspruch 1, **dadurch gekennzeichnet, dass** die ringförmige Schulter (16) zentral gegenüber einer kreisförmigen Öffnung der runden Kapillare (12) der Einlasskontur (8) ausgebildet ist.

3. Tropfenspender (1) nach Anspruch 1 oder 2, **dadurch gekennzeichnet, dass** die von der Kreisform abweichende Formgebung der Auslasskontur (9) nach Art einer Ellipse oder eines Polygons ausgebildet ist.

4. Tropfenspender (1) nach einem der Ansprüche 1 bis 3, **dadurch gekennzeichnet, dass** die numerische Exzentrizität der Auslasskontur (9) im Bereich 0,01 bis 0,8 liegt.

5. Tropfenspender (1) nach einem der Ansprüche 1 bis 4, **dadurch gekennzeichnet, dass** die gebildete Abtropfstufe einen Flächenbereich von 1 bis 7 mm² einnimmt.

6. Tropfenspender (1) nach einem der Ansprüche 1 bis 5, **dadurch gekennzeichnet, dass** eine Wandstärke einer die Auslassöffnung (6) aufnehmende Abgabespitze im Bereich der Auslassöffnung (6) zwischen Innenwandung und Außenwandung im Bereich 1:1 bis 1:6, insbesondere 1:1 bis 1:3, liegt.

7. Tropfenspender (1) nach einem der Ansprüche 1 bis 6, **dadurch gekennzeichnet, dass** der mittlere Ventilkörper (10) kopfseitig als gekrümmte Fläche ausgebildet ist.

8. Tropfenspender (1) nach Anspruch 7, **dadurch gekennzeichnet, dass** die gekrümmte Fläche kalottenartig ausgebildet ist.

9. Tropfenspender (1) nach Anspruch 7 oder 8, **dadurch gekennzeichnet, dass** die gekrümmte Fläche hydrophil ausgebildet ist.

10. Tropfenspender (1) nach einem der Ansprüche 1 bis 9, **dadurch gekennzeichnet, dass** die Auslasskontur (9) außenseitig eine Tropfenbildungsfläche (17) aufweist, die von einer Außenseite eines Tropfenbildungskörpers des Tropfaufsatzes (3) gebildet ist.

11. Tropfenspender (1) nach einem der Ansprüche 1 bis 10, **dadurch gekennzeichnet, dass** die Tropfenbildungsfläche (17) in einem Außenbereich bezogen auf eine Ausrichtung des Tropfenspenders (1) mit vertikal nach unten weisender Auslassöffnung (6) eine sich nach unten verjüngende Formgebung aufweist.

12. Tropfenspender (1) nach Anspruch 10 oder 11, **dadurch gekennzeichnet, dass** die Tropfenbildungsfläche (17) außenseitig durch eine Grenzkante begrenzt ist.

13. Tropfenspender (1) nach einem der Ansprüche 10 bis 12, **dadurch gekennzeichnet, dass** die Tropfenbildungsfläche (17) konkav oder konvex ausgebildet ist.

14. Tropfenspender (1) nach einem der Ansprüche 1 bis 13, **dadurch gekennzeichnet, dass** mindestens zwei mit axialem Abstand voneinander angeordnete ringförmige Schultern (16) vorgesehen sind.

15. Tropfenspender (1) nach einem der Ansprüche 1 bis 14, **dadurch gekennzeichnet, dass** das der Auslassöffnung (6) zugeordnete Auslassventil (7) mit einem vorgespannten Ventilkörper (10) ausgebildet ist.

16. Tropfenspender (1) nach Anspruch 15, **dadurch gekennzeichnet, dass** der Ventilkörper (10) mittels einer Ventilfeder (20) in Richtung der Auslassöffnung (6) kraftbeaufschlagt ist.

17. Tropfenspender (1) nach einem der Ansprüche 1 bis 16, **dadurch gekennzeichnet, dass** der Flüssigkeitsspeicher (2) als Quetschflasche ausgebildet ist und an einem Halsansatz der Quetschflasche der Tropfaufsatz (3) festlegbar ist.

18. Tropfenspender (1) nach einem der Ansprüche 1 bis 17, **dadurch gekennzeichnet, dass** ein Druckausgleichskanal (D) gegenüber dem Flüssigkeitsspeicher (2) und dessen Innenraum mittels einer Dichtvorrichtung abgedichtet ist.

19. Tropfenspender (1) nach Anspruch 18, **dadurch gekennzeichnet, dass** die Dichtvorrichtung als Dichtscheibe (21) oder Dichtstopfen ausgebildet ist.

20. Tropfenspender (1) nach einem der Ansprüche 1 bis 19, **dadurch gekennzeichnet, dass** der Tropfaufsatz (3) als Außenbauteil einen Abgabeadapter mit einem tubusförmigen Fortsatz umfasst, der ein Tropfadapter für die Ausgabe von Augentropfen ist.

21. Tropfenspender (1) nach einem der Ansprüche 1 bis 20, **dadurch gekennzeichnet, dass** ein Flüssigkeitsspeicher (2) zur Aufnahme des flüssigen Mediums zur Mehrfachverwendung vorgesehen ist.

## Revendications

1. Compte-gouttes (1) pour distribuer un liquide pharmaceutique formant une goutte, comprenant un réservoir de liquide (2) et une tête de compte-gouttes (3), dans lequel la tête de compte-gouttes (3) comporte un canal (5) se terminant en un orifice de sortie (6), dans lequel une soupape de sortie (7) est associée à l'orifice de sortie (6), et l'orifice de sortie (6) a une forme d'orifice capillaire avec un contour d'entrée (8) et un contour de sortie (9) et le contour d'entrée (8) forme un orifice annulaire intérieur (18) sur le côté sortie d'un corps de soupape central (10) de la soupape de sortie (7), dans lequel le capillaire (12) de l'orifice de sortie (6) est divisé par au moins un épaulement annulaire s'étendant radialement vers l'extérieur (16), **caractérisé en ce que** par rapport audit épaulement annulaire (16) le contour de sortie (9) s'étend avec un galbe s'écartant d'un galbe circulaire, et l'épaulement annulaire (16) forme un gradin d'égouttage à paroi intérieur avec des largeurs radiales circonférentiellement différentes (B1, B2) par rapport à l'ouverture annulaire (18) dans le contour d'entrée (8).

2. Compte-gouttes (1) selon la revendication 1, **caractérisé en ce que** l'épaulement annulaire (16) est formé au centre à l'opposée d'une ouverture circulaire dudit capillaire rond (12) du contour d'entrée (8).

3. Compte-gouttes (1) selon la revendication 1 ou 2, **caractérisé en ce que** le façonnage du contour de sortie (9) s'écartant du galbe circulaire est conçu à la manière d'une ellipse ou d'un polygone.

4. Compte-gouttes (1) selon l'une quelconque des revendications 1 à 3, **caractérisé en ce que** l'excentricité numérique du contour de sortie (9) est dans la plage de 0,01 à 0,8.

5. Compte-gouttes (1) selon l'une quelconque des revendications 1 à 4, **caractérisé en ce que** l'étage d'égouttage formé occupe une plage d'aire surfacique de 1 à 7 mm².

6. Compte-gouttes (1) selon l'une quelconque des revendications 1 à 5, **caractérisé en ce qu'**une épaisseur de paroi d'une pointe de distribution recevant l'ouverture de sortie (6) dans la région du contour extérieur entre la paroi intérieure et la paroi extérieure est dans la plage de 1:1 à 1:6, en particulier de 1:1 à 1:3.

7. Compte-gouttes (1) selon l'une quelconque des revendications 1 à 6, **caractérisé en ce que** le corps de soupape central (10) est conçu comme une surface courbée sur le côté tête.

8. Compte-gouttes (1) selon la revendication 7, **caractérisé en ce que** la surface courbée est façonnée en cuvette.

9. Compte-gouttes (1) selon la revendication 7 ou 8, **caractérisé en ce que** la surface courbée est hydrophile.

10. Compte-gouttes (1) selon l'une quelconque des revendications 1 à 9, **caractérisé en ce que** le contour de sortie (9) présente sur l'extérieur une surface de formation de goutte (17) formée par un extérieur d'un corps de formation de goutte de la tête de compte-gouttes (3).

11. Compte-gouttes (1) selon l'une quelconque des revendications 1 à 10, **caractérisé en ce que** la surface de formation de goutte (17) a un galbe se rétrécissant vers le bas dans une région extérieure par rapport à une orientation du distributeur de compte-gouttes (1) avec l'orifice (6) dirigé verticalement vers le bas.

12. Compte-gouttes (1) selon la revendication 10 ou 11, **caractérisé en ce que** la surface de formation de goutte (17) est délimitée sur l'extérieur par une arête de délimitation.

13. Compte-gouttes (1) selon l'une quelconque des revendications 10 à 12, **caractérisé en ce que** la surface de formation de goutte (17) est concave ou convexe.

14. Compte-gouttes (1) selon l'une quelconque des revendications 1 à 13, **caractérisé en ce qu'**il est prévu au moins deux épaulements annulaires (16) agencés à une distance axiale l'un de l'autre.

15. Compte-gouttes (1) selon l'une quelconque des revendications 1 à 14, **caractérisé en ce que** la soupape de sortie (7) associée à l'orifice de sortie (6) est formée avec un corps de soupape (10) précontraint.

16. Compte-gouttes (1) selon la revendication 15, **caractérisé en ce que** le corps de soupape (10) est sollicité par une force au moyen d'un ressort de soupape (20) en direction de l'ouverture de sortie (6).

17. Compte-gouttes (1) selon l'une quelconque des revendications 1 à 16, **caractérisé en ce que** le réservoir de liquide (2) est conçu comme un flacon compressible et la tête de compte-gouttes (3) est fixée sur une attache de col du flacon compressible.

18. Compte-gouttes (1) selon l'une quelconque des revendications 1 à 17, **caractérisé en ce qu'**un canal d'équilibrage de pression (D) est rendu étanche par rapport au réservoir de liquide (2) et à son intérieur au moyen d'un dispositif d'étanchéité.

19. Compte-gouttes (1) selon la revendication 18, **caractérisé en ce que** le dispositif d'étanchéité est conçu comme un disque d'étanchéité (21) ou un bouchon d'étanchéité.

20. Compte-gouttes (1) selon l'une quelconque des revendications 1 à 19, **caractérisé en ce que** la tête de compte-gouttes (3) comprend, en tant que composant externe, un adaptateur de distribution ayant un prolongement tubulaire qui est un adaptateur de compte-gouttes destiné à distribuer des gouttes ophtalmiques.

21. Compte-gouttes (1) selon l'une quelconque des revendications 1 à 20, **caractérisé en ce qu'**il possède un réservoir de liquide (2) destiné à recevoir le milieu liquide à usage multiple.
